(19) Europäisches Patentamt
European Patent Office
Office européen des brevets

(11) Veröffentlichungsnummer: **0 300 001 B1**

(12) **EUROPÄISCHE PATENTSCHRIFT**

(45) Veröffentlichungstag der Patentschrift: **28.10.92**

(51) Int. Cl.$^5$: **C07D 277/68**, C07D 277/64, C07K 5/06, C07K 5/08, C07K 5/10, C07H 17/00, G01N 33/535

(21) Anmeldenummer: **88901037.7**

(22) Anmeldetag: **14.01.88**

(86) Internationale Anmeldenummer:
**PCT/EP88/00024**

(87) Internationale Veröffentlichungsnummer:
**WO 88/05434 (28.07.88 88/17)**

(54) **AMINOLUCIFERINE, VERFAHREN ZU DEREN HERSTELLUNG UND IHRE VERWENDUNG.**

(30) Priorität: **14.01.87 DE 3700908**

(43) Veröffentlichungstag der Anmeldung:
**25.01.89 Patentblatt 89/04**

(45) Bekanntmachung des Hinweises auf die Patenterteilung:
**28.10.92 Patentblatt 92/44**

(84) Benannte Vertragsstaaten:
**AT BE CH DE FR GB IT LI LU NL SE**

(56) Entgegenhaltungen:
**EP-A- 0 024 525**
**WO-A-87/02667**

**JOURNAL OF THE AMERICAN CHEMICAL SOCIETY, Band 88, Nr. 9, 5. Mai 1966, Washington DC, US; E.H. WHITE et al.: "Amino analogs of firefly luciferin and biological activity thereof"**

(73) Patentinhaber: **Geiger, Reinhard, Dr.**
**Eisenhartstrasse 6**
**W-8000 München 60(DE)**

Patentinhaber: **Miska, Werner, Dr.**
**Haylerstrasse 8**
**W-8000 München 50(DE)**

(72) Erfinder: **Geiger, Reinhard, Dr.**
**Eisenhartstrasse 6**
**W-8000 München 60(DE)**
Erfinder: **Miska, Werner, Dr.**
**Haylerstrasse 8**
**W-8000 München 50(DE)**

(74) Vertreter: **Köster, Hajo, Dr. et al**
**Jaeger, Steffens & Köster Patentanwälte**
**Pippinplatz 4a**
**W-8035 München-Gauting(DE)**

EP 0 300 001 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Aminoluciferine, Verfahren zu deren Herstellung und deren Verwendung bei der Bestimmung von Enzymaktivitäten.

Bei den herkömmlichen analytischen Bestimmungen von Enzymaktivitäten, beispielsweise in biologischem Material, bedient man sich fluorogener oder chromogener Substrate, aus denen dann mit Hilfe der Enzyme eine "austretende Gruppe" freigesetzt werden kann.

Bei dieser austretenden Gruppe kann es sich beispielsweise um Farbstoffe handeln. Die bei dieser "Indikatorreaktion" entstehenden Farbstoffe sind der Fotometrie zugänglich. Daher ist es möglich, ihre Konzentration zu bestimmen und aus den gewonnenen Daten Rückschlüsse auf die enzymatische Aktivität zu ziehen, "Methods of Enzymatic Analysis", Verlag Chemie, Vol. 1-11, Weinheim, Bergstraße (1984), Hrsg. H.U. Bergmeyer.

Als Beispiel für eine derartige austretende Gruppe, deren Konzentration fotometrisch bestimmt werden kann, sei die folgende genannt:

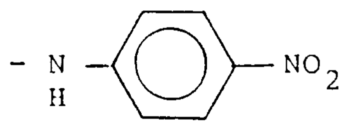

Setzt man ein oben genanntes fluorogenes Substrat ein, dann kann man die austretende Gruppe fluorometrisch bestimmen. Als Beispiel für die austretende Gruppe eines fluorogenen Substrats sei die folgende genannt:

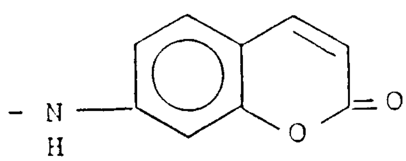

Mit den bisher bekannten Substraten können Enzym-Konzentrationen bis ca. 5 ng pro Test gemessen werden. Es handelt sich hier um die enzymatische Aktivität (U). Aus der spezifischen Aktivität (U/mg) kann auf die Menge an Enzym geschlossen werden.

Die enzymatische Aktivität (U) ist definiert als µmol/min.: meist bei 25 °C gemessen. Diese enzymatische Aktivität, die auch katalytische Aktivität genannt wird, wird in SI-einheiten in Katal (kat = mol/s) angegeben. Die katalytische Konzentration (katalytische Aktivität pro Volumen) wird in kat/l angegeben.

Erfindungsgemäß werden nun neue Substrate bereitgestellt, mit denen die Empfindlichkeit der eingangs genannten analytischen ßestimmungsmethoden von Enzymaktivitäten überraschenderweise erheblich gesteigert werden kann. So können Enzymkonzentrationen bis zu ca. 10 bis 100 fg pro Test gemessen werden. Dies heißt, daß die Nachweisgrenze nach "unten" verschoben wird. Die Empfindlichkeit hängt dabei natürlich vom untersuchten Enzym und dem eingesetzten Substrat ab.

Allen erfindungsgemäß bereitgestellten Substraten ist gemeinsam, daß aus ihnen durch die untersuchten Enzyme D-Aminoluciferin der Formel (V)

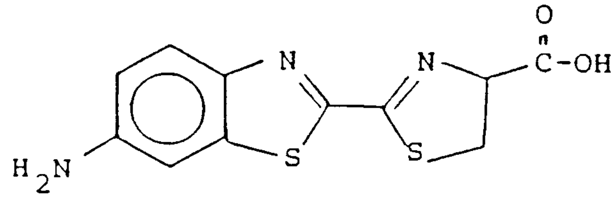

(V)

freigesetzt wird.

Das Aminoluciferin (V) stellt somit eine austretende Gruppe (leaving group) dar, wie dies eingangs geschildert wurde.

Das freigesetzte Aminoluciferin ist noch in geringster Konzentration luminometrisch nachweisbar. Dazu setzt man das Aminoluciferin mit dem Enzym Luciferase des Leuchtkäfers Photinus pyralis oder des Leuchtkäfers Photinus plathiophthalamus oder mit der Luciferase anderer Species oder mit chemisch oder genetisch modifizierten Luciferasen in Gegenwart von ATP + $MgCl_2$ um. Bei dieser Reaktion werden Photonen emittiert; und zwar bei der Umsetzung mit dem Enzym des Leuchtkäfers Photinus pyralis bei 605 nm und bei der Umsetzung mit dem Enzym des Leuchtkäfers Photinus plathiophthalamus bei 549 bzw. 570 nm oder bei der dem eingesetzten Luciferin/Luciferase-System entsprechenden Wellenlänge. Die Emission bei 549 nm findet statt, wenn das Enzym aus dem dorsalen Organ des genannten Leuchtkäfers stammt, während die Emission bei 570 nm stattfindet, wenn das Enzym aus dem ventralen Organ stammt.

Es handelt sich dabei um eine Biolumineszenz. Das emittierte Licht bestimmt man luminometrisch.

Für weitere Einzelheiten wird auf folgende Literaturstellen verwiesen:

"Luminometry" von K. Wulff in "Methods of Enzymatic Analysis", Vol. 1 (Hrsg. H.U. Bergmeyer), Seiten 340-368, Verlag Chemie, Weinheim, Bergstraße (1983) und Journal of the American Chemical Society 88, 2015-2018 (1966)von E.H. White et al.

Aus den so erhaltenen Daten kann man die Enzymkonzentration des untersuchten Enzyms bestimmen. Die genaue Durchführung dieser Bestimmung mit Hilfe eines "Biolumineszenz-Cocktails" sowie die Eichung sind in den Beispielen näher erläutert.

Gegenstand der Erfindung sind somit D-Aminoluciferine der allgemeinen Formel (I)

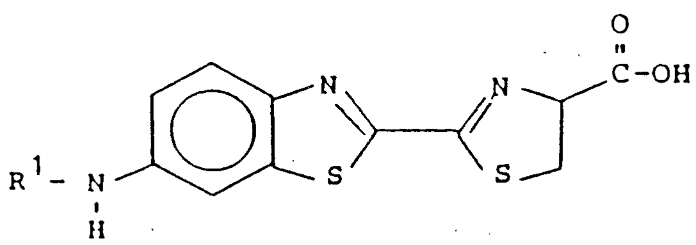

(I)

worin

$R^1$ einen L-Aminosäure- oder Peptidrest mit bis zu 10 L-Aminosäureeinheiten, der über die (endständige) Carboxylgruppe als Amid gebunden ist und dessen freie Aminogruppe(n) gegebenenfalls durch eine übliche Schutzgruppe geschützt sind, oder einen Monosaccharid- oder Disaccharidrest bedeutet.

Die erfindungsgemäßen Aminosäurereste sind über die Carboxygruppe an die Aminogruppe des Luciferins in Form eines Amids gebunden. Es handelt sich dabei um Aminosäuren mit L-Konfiguration. Die Aminogruppe befindet sich in $\alpha$-Stellung zur Carboxylgruppe.

Die Aminosäurereste bzw. -einheiten stammen vorzugsweise von natürlich vorkommenden Aminosäuren.

Der Aminosäurerest $R^1$ leitet sich von üblichen Aminosäuren ab, beispielsweise

Aminosäuren mit unpolarem Rest, z.B. Glycin, Alanin, Valin, leucin, Isoleucin, Prolin und Phenylalanin,

solchen mit nicht ionisierten, aber polar wirkenden, Gruppen, z.B. Tyrosin, Tryptophan, Serin, Threonin, Cystein, Cystin, Methionin, Hydroxyprolin, Allothreonin, Homoserin und Homocystein,

sauren Aminosäuren (Aminodicarbonsäuren ), z.B. Asparagin- und Glutaminsäure (und auch die Amide davon, i.e. Asparagin und Glutamin),

und basischen Aminosäuren, z.B. Lysin, Arginin und Histidin.

Der Rest $R^1$ kann sich auch von Hydroxylysin, $\alpha$-Aminoadipinsäure, Ornithin, Citrullin, Homoarginin, $\alpha$, $\epsilon$ -Diaminopimelinsäure, $\gamma$-Aminobuttersäure und Phenylserin ableiten.

Vorzugsweise entspricht der Aminosäurerest dar allgemeinen Formel (II)

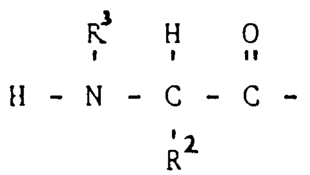

(II)

worin
$R^3$ ein Wasserstoffatom oder eine übliche Schutzgruppe darstellt und
$R^2$ für -H, $-CH_3$, $-CH_2OH$, $-CH(OH)CH_3$, $-CH(CH_3)_2$, $-CH_2CH(CH_3)_2$, $-CH(CH_3)CH_2CH_3$ , $-CH_2COOH$, $-CH_2CONH_2$, $-CH_2CH_2COOH$, $-CH_2CH_2CONH_2$, $-CH_2CH_2CH_2CH_2NH_2$, $-CH_2C_6H_4OH$, $-CH_2C_6H_5$,

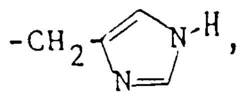, 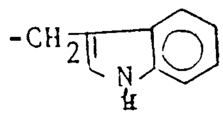 ,

$-CH_2SH$, $-CH_2cH_2CH_2$-NH-C(=NH)-$NH_2$ oder $-CH_2CH_2SCH_3$ steht.

Zu den bevorzugten Resten zählen auch die Prolin- und Hydroxyprolinreste.

Weiterhin bevorzugte Reste $R^2$ sind: $-CH_2C_6H_5$, $-CH_2CH_2CH_2$-NH-C(=NH)-$NH_2$,$-CH_2C_6H_4OH$, $-CH_2CH_2CH_2CH_2NH_2$ oder $-CH_3$.

Als Aminoschutzgruppe kann man jede übliche Schutzgruppe einsetzen. Bevorzugt sind die Acetyl-, Benzoyl-, Tosyl-, Benzyloxycarbonyl-, tert.-Butyloxycarbonyl-, Succinyl- oder Methoxysuccinyl-Schutzgruppen.

Der Peptidrest der erfindungsgemäßen Verbindungen der allgemeinen Formel (I) setzt sich aus beliebigen der og. Aminosäuren zusammen und enthält bis zu zehn derartige Aminosäure-Einheiten. Der Peptidrest ist über die endständige Carboxygruppe an den Luciferin-Grundkörper gebunden.

Dieser Peptidrest weist vorzugsweise fünf Aminosäure-Einheiten auf. Es handelt sich insbesondere bevorzugt um einen Peptidrest mit zwei Aminosäure-Einheiten.

Bedeutet $R^1$ bei den erfindungsgemäßen Verbindungen der allgemeinen Formel (I) einen Monosaccharidrest, dann leitet sich dieser von einer üblichen Hexose oder Pentose ab. Dazu zählen beispielsweise Glucose, Galaktose, Mannose, Fucose, Ribose, Desoxyribose, Fructose und Lactose. Diese sind über $C^1$ an den Luciferin-Grundkörper gebunden.

Der Disaccharidrest setzt sich aus den genannten Zuckerresten zusammen.

Gegenstand der Erfindung ist auch ein Verfahren zur Herstellung der D-Aminoluciferine der allgemeinen Formel (I). Dieses Verfahren ist dadurch gekennzeichnet, daß man

a) zur Herstellung der Verbindungen der allgemeinen Formel (I), worin $R^1$ einen wie zuvor definierten L-Aminosäure- oder Pentidrest bedeutet,
eine (ein ) dem zuvor definierten Aminosäure- und Peptidrest entsprechende(s) N-geschützte(s) Aminosäure bzw. Peptid
entweder mit 2-Cyano-6-aminobenzothiazol der Formel (III)

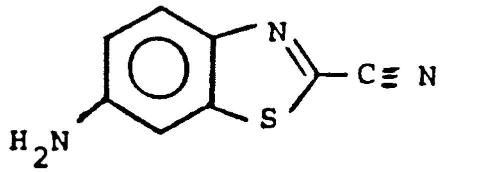 (III)

zu einer Verbindung der allgemeinen Formel (IV)

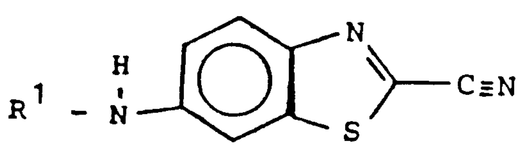 (IV)

umsetzt, worin $R^1$ die oben angegebenen Bedeutungen besitzt, und
die erhaltene Verbindung der allgemeinen Formel (IV) mit D-Cystein zu einer Verbindung der allgemeinen Formel (I) umsetzt und
gegebenenfalls die Aminoschutzgruppe(n) auf übliche Weise abspaltet
oder

mit Aminoluciferin, dessen Carboxylgruppe durch eine übliche Schutzgruppe geschützt ist, der Formel (Va)

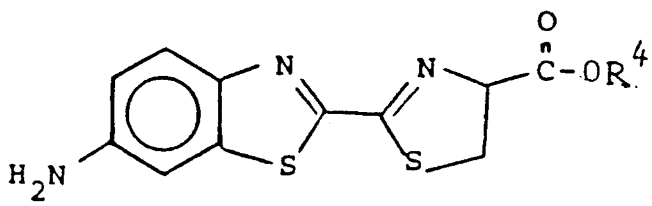

(Va)

worin

$R^4$ eine übliche Carboxylschutzgruppe bedeutet, umsetzt und

die Carboxylschutzgruppe sowie gegebenenfalls die Aminoschutzgruppe(n) auf übliche Weise entfernt, oder

b) zur Herstellung einer Verbindung der allgemeinen Formel (I), worin $R^1$ einen Monosaccharid- oder Disaccharidrest bedeutet,

Aminoluciferin der Formel (Va), dessen Carboxylschutzgruppe geschützt ist, mit dem entsprechenden $Br^1$-Mono- oder Disaccharid umsetzt und anschließend die Schutzgruppe der Carboxylgruppe abspaltet.

Die Verbindung (III) setzt man zu der Verbindung (IV) in Anwesenheit von Phosphortrichlorid um. Man arbeitet dabei zweckmäßigerweise in einem reaktionsinerten absoluten Lösungsmittel, z.B. in THF, DMF oder DMSO, vorzugsweise in wasserfreiem Pyridin und unter Rühren bei niedrigen Temperaturen, vorzugsweise bei -5 ° C bis -20 ° C. Bei dieser Umsetzung handelt es sich um die Phospho-azo-Methode.

Man kann die Verbindungen (IV) aus der Verbindung (III) auch nach der gemischten Anhydrid-Methode herstellen.

Dazu löst man die N-geschützte Aminosäure oder das N-geschützte Peptid in einem reaktionsinerten Lösungsmittel, vorzugsweise absolutem Pyridin, THF, DMF oder DMSO und versetzt diese Lösung mit Chlorameisensäure-isobutylester und einem tertiären Amin, vorzugsweise Triethylamin. Zu dieser Lösung gibt man die Verbindung (III).

Die Umsetzung der Verbindung (IV) mit D-Cystein zum Aminoluciferin nimmt man vorzugsweise in $N_2$-gesättigtem Wasser vor.

Geht man von einem geschützten Aminoluciferin der Formel (Va) aus, dann kann die Aminosäure (oder das Peptid) ebenfalls sowohl mittels der Phospho-azo-Methode als auch mittels der gemischten Anhydrid-Methode einführen. Die Reaktionsbedingungen entsprechen dabei den oben angegebenen.

Die Aminoschutzgruppen kann man auf bekannte Weise entfernen, beispielsweise durch katalytische Hydrierung mit beispielsweise Palladium-auf-Kohle in Methanol, durch Hydrazinolyse oder durch eine milde Säurebehandlung.

Als Carboxylschutzgruppen setzt man vorzugsweise eine Methyl-, Ethyl-, tert.-Butyl-, Benzyl- oder Phenacylgruppe ein. Diese kann man auf bekannte Weise entfernen, beispielsweise durch basenkatalysierte Hydrolyse. Im Falle des Benzylesters kann man auch mit Palladium-auf-Kohle umsetzen. Vorzugsweise schützt man die Carboxylgruppe dadurch, daß man sie in den Methylester überführt. Diesen spaltet man in freie Säure und Methanol, vorzugsweise durch Behandeln mit Carboxylesterase.

Die Aminosäure (oder das Peptid) kann somit an 2-Cyano-6-aminobenzothiazol (III) oder an Aminoluciferin nach zwei Methoden gebunden werden, nämlich mittels der Phospho-azo-Methode und mittels der "gemischten Anhydrid-Methode". Diese Umsetzungen sind in den nachstehenden Schemata 1 und 2 näher erläutert.

Schema 1

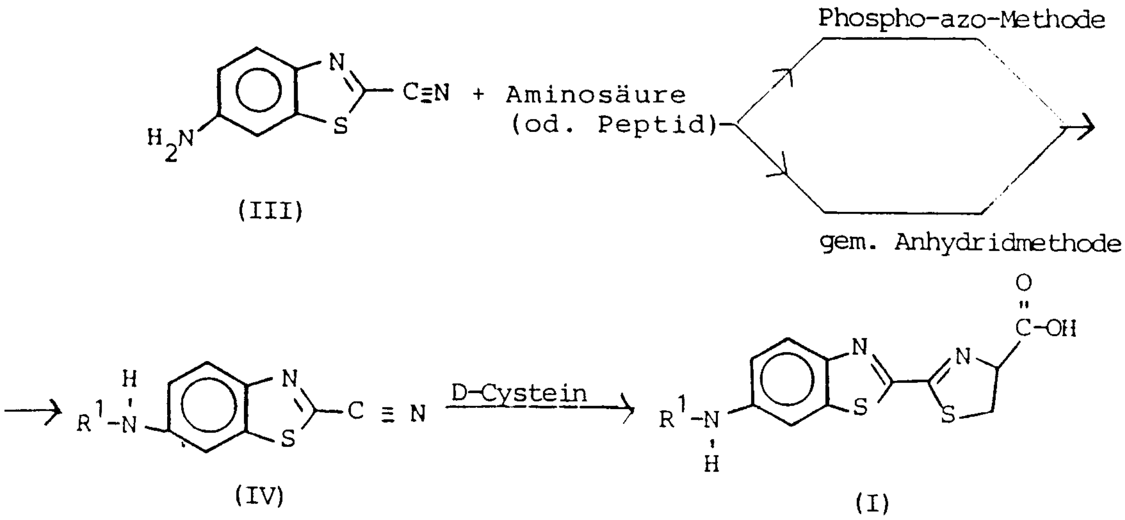


(III) (IV) (I)

Schema 2

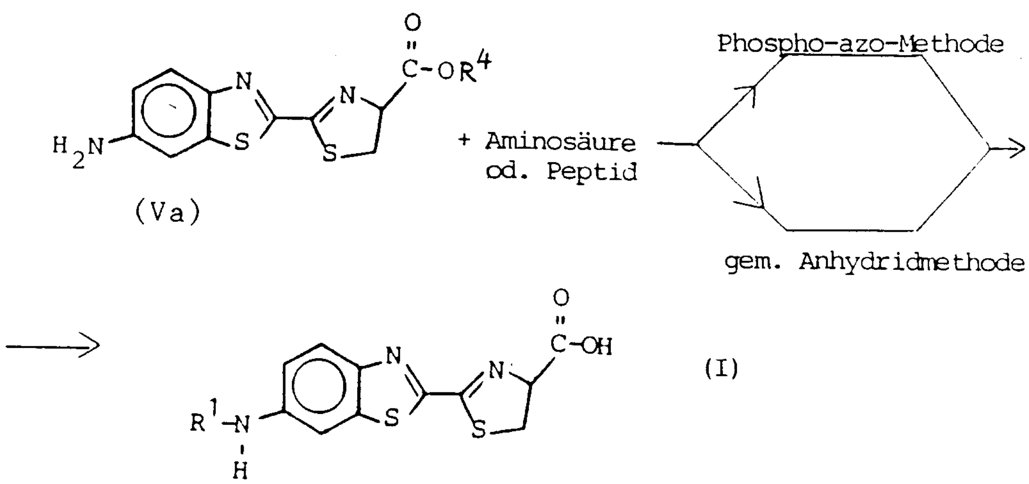


(Va) (I)

Das Aminoluciferin stellt man nach in der Literatur beschriebenen Verfahren wie folgt her:

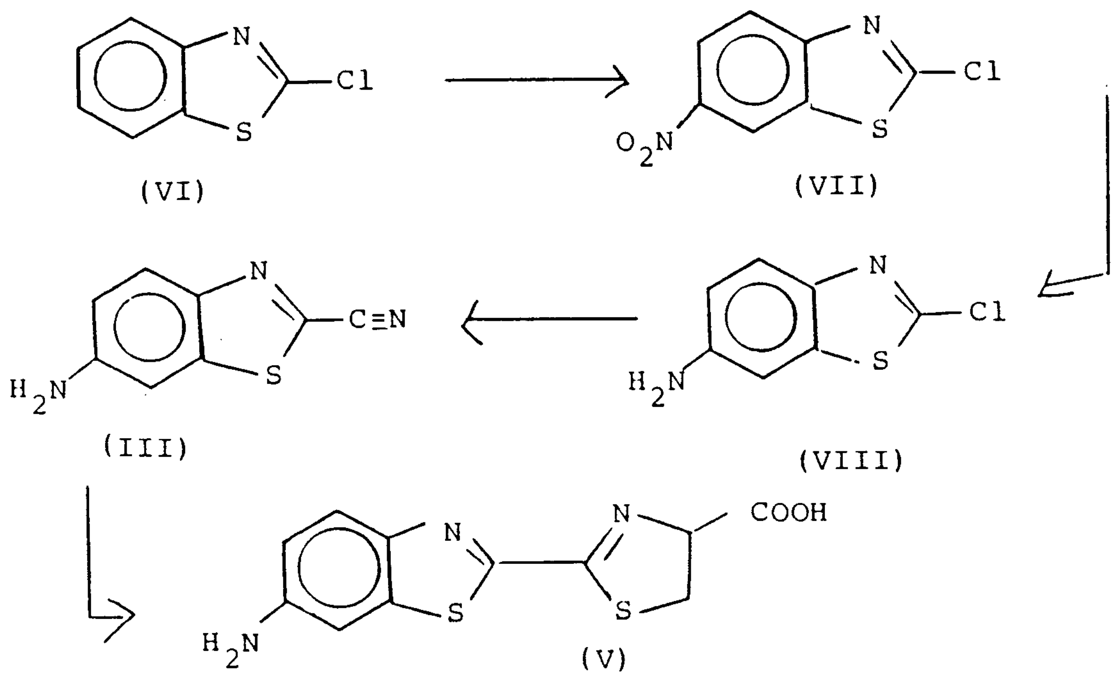

(VI) (VII) (VIII) (III) (V)

VI: im Handel erhältlich
VII und VIII: dargestellt nach Katz, J.Am.Chem.Soc. 73, 4007 (1951)
III und V: dargestellt nach White et al., J.Am. Chem. Soc. 88, 2015 (1966)

Es ist zu betonen, daß erfindungsgemäß sowohl das freie Aminoluciferin als auch die Aminosäurederivate ($R^1$ = angegebene Bedeutungen) D-Konfiguration besitzen.

Auch bei der Herstellung der erfindungsgemäßen Verbindungen der allgemeinen Formel (I), worin $R^1$ einen Mono- oder Disaccharidrest bedeutet, geht man vom Aminoluciferin aus. Man schützt die Carboxygruppe des Aminoluciferins, indem man sie beispielsweise methyliert. Diese geschützte Verbindung setzt man dann mit einem $Br^1$-Mono- oder Disaccharid um, und spaltet anschließend die Methylgruppe mit Carboxylesterase ab.

Mit den erfindungsgemäßen Verbindungen kann die Enzymaktivität der verschiedensten Enzyme bestimmt werden. Diese Enzyme müssen in der Lage sein, das Aminoluciferin (V) aus den erfindungsgemäßen Verbindungen freizusetzen, d. h. sie müssen in der Lage sein, den Rest $R^1$ abzuspalten. Diese Enzyme müssen daher die Amidbindung zwischen dem Aminoluciferin und dem Aminosäurerest spalten.

Der Fachmann weiß, daß Enzyme mehr oder weniger spezifisch sind hinsichtlich der Amidbindung und hinsichtlich der Aminosäure. Einige Enzyme sind beispielsweise lediglich in der Lage, die Amidbindung von ganz bestimmten Aminosäuren zu spalten.

Wünscht man ein vorgegebenes Enzym zu bestimmen, dann geht man zweckmäßigerweise wie folgt vor:
Ist bekannt, daß dieses Enzym lediglich in der Lage ist, die Amidbindung spezifischer Aminosäuren zu spalten, dann setzt man ein erfindungsgemäßes Aminoluciferin ein, das als Rest $R^1$ diese Aminosäureeinheit aufweist, mit der das Enzym spezifisch reagiert.

Nachstehend wird der erfindungsgemäß eingesetzte luminometrische Test näher erläutert:

Für diesen luminometrischen Test verwendet man einen "Biolumineszens-Cocktail" mit folgender Zusammensetzung:

30 mmol/l HEPES (N-2-Hydroxyethylpiperazin-N'-2-ethansulfonsäure)

6 mmol/l $MgCl_2$

6 mmol/l ATP

0,5mmol/l EDTA

80 µmol/l DTT (Dithiothreitol)

1 µg Luciferase (des Leuchtkäfers Photinus pyralis oder plathiophthalamus oder von anderen Species

Gesamtvolumen: 0,4 ml; pH 7,75

Um eine unbekannte Enzymkonzentration bestimmen zu können, ist es erforderlich, eine Eichkurve zu erstellen. Dazu versetzt man Lösungen mit unterschiedlichen, jedoch bekannten Konzentrationen an Aminoluciferin mit einer bestimmten Menge des Biolumineszenz-Cocktails und mißt dann die Zahl der Lichtimpulse während eines Zeitraums von 10 sec.

Einer bestimmten Anzahl an Lichtimpulsen ist somit eine bestimmte Aminoluciferin-Konzentration zugeordnet. Bei der Bestimmung einer unbekannten Enzymaktivität wird eine bestimmte Menge Aminoluciferin aus den erfindungsgemäßen Verbindungen der allgemeinen Formel (I) freigesetzt. Diese Menge an freigesetztem Aminoluciferin läßt sich mit Hilfe des luminometrischen Tests bestimmen. Man weiß dann, welche Menge an Aminoluciferin freigesetzt wurde und kann daraus auf die Enzymaktivität rückschließen.

Bei der Durchführung dieses luminometrischen Tests versetzt man zweckmäßigerweise 0,1 ml des Testansatzes mit 0,4 ml des Biolumineszenz-Cocktails und mißt dann 10 sec. lang die Lichtimpulse.

Nachstehend sind verschiedene Tests zur Bestimmung unterschiedlicher Enzymaktivitäten beschrieben.

Beispiel 1

Bestimmung von Chymotrypsin und Chymotrypsin-ähnlicher Enzymaktivität, beispielsweise in Faeces. Als erfindungsgemäße Verbindung setzt man $N^{\alpha}$-Acetyl-L-phenyl-alanyl-amino-luciferin ein.

Man stellt folgenden Testansatz her:

0,90 ml Puffer (0,2 mol/l Triethanolamin, 0,02 mol/l $CaCl_2$, pH = 7,8)

0,05 ml Substratlösung ($N^{\alpha}$-Acetyl-L-phenylalanyl-aminoluciferin; 1 mmol/l in $H_2O$)

Man inkubiert dann 5 min bei 25 °C. Anschließend gibt man

0,05 ml einer Chymotrypsin enthaltenden Lösung zu.

Nach genau einer Minute entnimmt man 0,1 ml des Testansatzes und versetzt mit 0,4 ml des oben beschriebenen Biolumineszenz-Cocktails und mißt dann 10 sec. lang die Lichtimpulse.

Bei dieser Bestimmung kann man das eingesetzte $N^{\alpha}$-Acetyl-L-phenylalanin -amino-luciferin durch die gleiche Menge an $N^{\alpha}$-Acetyl-L-tyrosyl-amino-luciferin ersetzen.

Mit diesem Test kann man eine Chymotrypsin-Menge bis zu 10 fg bestimmen. Durch Veränderungen der Versuchsbedingungen (Inkubationszeit im Test, Reaktionstemperatur) läßt sich die Nachweisgrenze noch weiter nach unten verschieben.

Beispiel 2

Bestimmung von Trypsin und Trypsin-ähnlicher enzymatischer Aktivität.

Als erfindungsgemäße Verbindung wird $N^{\alpha}$-Acetyl-L-arginyl-amino-luciferin eingesetzt.

Der Testansatz besitzt folgende Zusammensetzung:

0,75 ml Puffer (0,2 mol/l Triethanolamin, 0,02 mol/l $CaCl_2$; pH = 7,8)

0,05 ml Substratlösung (1 mmol/l $N^{\alpha}$-Acetyl-L-arginyl-aminoluciferin in $H_2O$)

Man inkubiert 5 min bei 25 °C und gibt dann

0,2 ml Trypsinlösung zu.

Nach genau einer Minute entnimmt man 0,1 ml des Testansatzes und versetzt mit 0,4 ml des Biolumineszenz-Cocktails. Anschließend führt man den luminometrischen Test wie oben beschrieben durch.

Mit diesem Test läßt sich eine Trypsin-Menge bis zu 10 fg bestimmen. Auch in diesem Fall kann durch Verändern der Versuchsbedingungen die Nachweisgrenze nach unten verschoben werden.

Bei diesem Test kann man ferner anstelle von $N^{\alpha}$-Acetyl-L-arginyl-amino-luciferin auch $N^{\alpha}$-Acetyl-L-

lysyl-amino-luciferin einsetzen.

Dieser Test kann auch zur Bestimmung von Kallikrein verwendet werden.

Beispiel 3

Bestimmung von Elastase und Elastase-ähnlicher Aktivität.

Als erfindungsgemäße Verbindung setzt man $N^{\alpha}$-Acetyl-L-alanyl-amino-luciferin ein.

Der Testansatz besitzt folgende Zusammensetzung:

0,85 ml Puffer (0,2 mol/l Triethanolamin; pH = 7,8)
0,05 ml Substratlösung (1 mmol/l $N^{\alpha}$-Acetyl-L-alanyl-aminoluciferin in $H_2O$)
Man inkubiert dann 5 min bei 25 °C und gibt
0,10 ml Elastase enthaltende Lösung zu.

Nach einer Minute entnimmt man 0,1 ml des Testansatzes und versetzt mit 0,4 ml des oben beschriebenen Biolumineszenz-Cocktails. Anschließend mißt man 10 sec. lang die Lichtimpulse.

Mit diesem Test läßt sich eine Elastase-Menge von bis zu 10fg bestimmen.

Herstellungsbeispiele

1. Herstellung der Aminoluciferine der allgemeinen Formel (I), worin $R^1$ einen Aminosäure- oder Peptidrest bedeutet.

a) Herstellung mittels der Phospho-azo-Methode ausgehend von 2-Cyano-6-aminobenzothiazol der Formel (III)

17,5 mg (0,1 mmol) 2-Cyano-6-aminobenzothiazol (III) löst man bei -10°C in wasserfreiem Pyridin und versetzt unter Rühren mit 5 µl (0,056 mmol) Phosphortrichlorid. Nach 1 h bei -10°C gibt man eine Suspension von 0,11 mmol geschützte(s) Aminosäure oder Peptid in eiskaltem Pyridin zum Reaktionsansatz. Man läßt langsam auf Raumtemperatur erwärmen und rührt 20 h bei RT. Anschließend zieht man Pyridin im Vakuum ab und nimmt den Rückstand in wassergesättigtem Butanol auf, filtriert und engt anschließend ein. Den Rückstand nimmt man in wenig Wasser auf und reinigt über HPLC (Lösung A).

0,11 mmol D-Cystein löst man in $N_2$-gesättigtem Wasser, stellt auf pH 7,5 ein und spült mit $N_2$. Dazu gibt man die zuvor mit Stickstoff gesättigte Lösung A und rührt 2 h unter Stickstoff lichtgeschützt bei Raumtemperatur. Anschließend wird die Lösung eingeengt und in Wasser/Ethanol 50/50 aufgenommen. Nachdem gegebenenfalls die Aminoschutzgruppe(n) auf übliche Weise entfernt worden sind, wird über HPLC gereinigt.

b) Darstellung mittels der "gemischten Anhydrid-Methode" ausgehend von 2-Cyano-6-benzothiazol der Formel (III)

0,135 mmol Aminosäure oder Peptid, gelöst in absolutem Tetrahydrofuran (DMF oder DMSO), kühlt man auf -15°C. Dann versetzt man die Lösung mit 14 µl (0,1 mmol) Triethylamin und 13 µl (0,1 mmol) Chlorameisensäure-isobutylester und rührt 30 Min bei -15°C. Danach gibt man 17,5 mg (0,1 mmol) 2-Cyano-4-amino-benzothiazol (III) in Dimethylformamid (vorgekühlt) zu, läßt langsam auf Raumtemperatur erwärmen und rührt über Nacht weiter. Anschließend filtriert man ausgefallenes Triethylammonium-hydrochlorid ab, engt das Filtrat ein und nimmt den Rückstand in wenig Wasser auf und reinigt über HPLC (Lösung B).

0,1 mmol D-Cystein löst man in $N_2$gesättigtem Wasser stellt auf pH 7,5 und spült mit $N_2$ weiter. Dazu gibt man die zuvor mit Stickstoff gesättigte Lösung B und rührt 2h unter Stickstoff lichtgeschützt bei Raumtemperatur. Anschließend engt man die Lösung ein, nimmt in Wasser/Ethanol (50/50) auf, spaltet gegebenenfalls die Aminoschutzgruppe(n) ab und reinigt über HPLC.

c) Herstellung mittels der Phospho-azo-Methode ausgehend von Aminoluciferin der Formel (V)

0,05 mmol geschütztes Aminoluciferin (Va) (vorzugsweise den Methylester) löst man bei -10 °C in wasserfreiem Pyridin und versetzt unter Rühren mit 2,5 µl (0,028 mmol) Phosphortrichlorid. Nach 1 h bei -10 °C gibt man 0,055 mmol geschützte(s) Aminosäure oder Peptid, suspendiert in eiskaltem Pyridin, zum Reaktionsansatz. Man läßt langsam auf Raumtemperatur erwärmen und rührt 20 h bei Raumtemperatur (RT).

Anschließend zieht man Pyridin im Vakuum im Rotationsverdampfer ab und nimmt den Rückstand in wassergesättigtem Butanol auf, filtriert und engt anschließend ein. Den Rückstand nimmt man in wenig Wasser auf, spaltet die Carboxylschutzgruppe (im Falle des Methylesters mit Hilfe von Carboxylesterase) sowie gegebenenfalls die Aminoschutzgruppe(n) ab und reinigt über HPLC.

d) Herstellung mittels der "gemischten Anhydrid-Methode" ausgehend von Aminoluciferin der Formel

(V)
0,0135 mmol geschützte(s) Aminosäure oder Petid, gelöst in absolutem Tetrahydrofuran (DMF, DMSO), kühlt man auf -15 °C. Dann versetzt man die Lösung mit 1,4 µl (0,01 mmol) Triethylamin und 1,3 µl (0,01 mmol) Chlorameisensäure-isobutylester und rührt 30 Min. bei -15 °C. Danach gibt man 0,01 mmol geschütztes Aminoluciferin (V) in Dimethylformamid (vorgekühlt) zu, läßt langsam auf Raumtemperatur erwärmen und rührt über Nacht weiter. Anschließend filtriert man ausgefallenes Triethylammonium-hydrochlorid ab, engt das Filtrat ein, nimmt den Rückstand in wenig Wasser auf und spaltet die Carboxylschutzgruppe sowie gegebenenfalls die Aminoschutzgruppe(n) anschließend reinigt man mittels HPLC.

Nach obigen Verfahrensweisen stellt man folgende Amino-luciferine her:

$N^{\alpha}$-Acetyl-L-phenylalanyl-amino-luciferin

Analyse für $C_{22}H_{20}N_4S_2O_4$ (M = 468,56)

| | C | H | N |
|---|---|---|---|
| berechnet: | 56,3 % | 4,3 % | 12,0 % |
| gefunden: | 56,2 % | 4,3 % | 12,1 % |

| Aminosäuregehalt: | |
|---|---|
| berechnet: | 35,21 % |
| gefunden: | 34,15 % |

$N^{\alpha}$-Acetyl-L-arginyl-amino-luciferin

Analyse für $C_{19}H_{23}N_7S_2O_4$ (M =477,58)

| | C | H | N |
|---|---|---|---|
| berechnet: | 47,7 % | 4,8 % | 20,5 % |
| gefunden: | 47,5 % | 4,7 % | 20,4 % |

| Aminosäuregehalt: | |
|---|---|
| berechnet: | 36,43 % |
| gefunden: | 35,70 % |

$N^{\alpha}$-Acetyl-L-tyrosyl-amino-luciferin

Analyse für $C_{22}H_{20}N_4S_2O_5$ (M =484,57)

| | C | H | N |
|---|---|---|---|
| berechnet: | 54,5 % | 4,1 % | 11,6 % |
| gefunden: | 54,6 % | 4,0 % | 11,6 % |

| Aminosäuregehalt: | |
|---|---|
| berechnet: | 44,78 % |
| gefunden: | 43,54 % |

N$^{\alpha}$-Acetyl-L-lysyl-amino-luciferin

Analyse for $C_{19}H_{23}N_5S_2O_4$ (M = 449,56)

| | C | H | N |
|---|---|---|---|
| berechnet: | 50,7 % | 5,1 % | 15,6 % |
| gefunden: | 50,6 % | 5,0 % | 15,8 % |

| Aminosäuregehalt: | |
|---|---|
| berechnet: | 32,48 % |
| gefunden: | 31,83 % |

N$^{\alpha}$-Acetyl-L-alanyl-amino-luciferin

Analyse für $C_{16}H_{16}N_4SO_4$ (M =360,38)

| | C | H | N |
|---|---|---|---|
| berechnet: | 53,3 % | 4,5 % | 15,6 % |
| gefunden: | 53,2 % | 4,5 % | 15,6 % |

| Aminosäuregehalt: | |
|---|---|
| berechnet: | 24,70 % |
| gefunden: | 23,96 % |

Nach den oben beschriebenen Arbeitsweisen wurden folgende Verbindungen hergestellt:

a) N$^{\alpha}$-Aetyl-L-phenylalanyl-L-arginylaminoluciferin ($C_{28}H_{32}N_8S_2O_5$ (M = 624,75))
b) N$^{\alpha}$-Methoxysuccinyl-L-alanyl-L-alanyl-L-valylaminoluciferin ($C_{27}H_{34}N_{62}SO_7$ (M = 618,74))
c) N$^{\alpha}$-Methoxysuccinyl-L-alanyl-L-alanyl-L-proly-L-valyl-aminoluciferin ($C_{32}H_{41}N_7S_2O_9$ (M = 731,85))
d) N$^{\alpha}$-Methoxysuccinyl-L-alanyl-L-alanyl-L-proly-L-phenylalanylaminoluciferin ($C_{36}H_{41}N_7S_2O_9$ (M = 779,90))
e) N$^{\alpha}$-Acetyl-L-alanyl-L-alanyl-L-alanylaminoluciferin ($C_{21}H_{26}N_6S_2O_6$) (M = 522,61))
f) N$^{\alpha}$-Acetyl-L-phenylalanyl-L-glycylaminoluciferin ($C_{24}H_{23}N_5S_{25}O$ (M = 525,61))
g) N$^{\alpha}$-Acetyl-L-glycyl-L-arginylaminoluciferin ($C_{21}H_{26}N_8S_2O_5$ (M = 534,62))
h) Pyroglutamyl-L-glycyl-L-arginylaminoluciferin ($C_{24}H_{29}N_9S_2O_6$ (M = 603,68))
i) Pyroglutamyl-L-phenylalanyl-L-leucylaminoluciferin ($C_{31}H_{34}N_6S_2O_6$ (M = 650,78))
j) Succinyl-L-alanyl-L-alanyl-L-prolyl-L-methionylaminoluciferin ($C_{31}H_{39}N_7S_3O_9$ (M = 707,87))
k) N$^{\alpha}$-Benzoyl-L-prolyl-L-phenylalanyl-L-arginylaminoluciferin ($C_{38}H_{41}N_9S_2O_6$ (mw = 783.93))
l) N$^{\alpha}$-Tosyl-glycyl-L-prolyl-L-lysylaminoluciferin ($C_{31}H_{37}N_6S_3O_7$ (mw = 701.87))
m) N$^{\alpha}$-Benzoyl-$\beta$-alanyl-glycyl-L-arginylaminoluciferin ($C_{29}H_{33}N_9S_2O_6$ (mw = 667.77))
n) N$^{\alpha}$-Carbobenzoxy-L-valyl-glycyl-L-arginylaminoluciferin ($C_{32}H_{38}N_9S_2O_8$ (mw = 740.84))
o) N$^{\alpha}$-Tosyl-glycyl-L-prolyl-L-arginylaminoluciferin ($C_{31}H_{37}N_9S_3O_7$ (mw = 743.89))

Die unter a), g) und h) aufgeführten Aminoluciferine werden vorzugsweise nach der gemischten Anhydrid-Methode hergestellt.

In der nachfolgenden Tabelle ist aufgeführt, welche Enzyme durch welche erfindungsgemäßen Aminoluciferine bestimmt werden können. Es ist somit aufgeführt, welche Enzyme in der Lage sind, das als Substrat eingesetzte Aminoluciferin-Derivat in das freie Aminoluciferin sowie in die entsprechende Aminosäure bzw. in das entsprechende Peptid zu spalten.

| Aminoluciferin | wird gespalten durch das Enzym |
|---|---|
| a) | Gewebskallikrein EC 3.4.21.35 |
| b) | Leukocytenelastase EC 3.4.21.37 |
| c) | Leukocytenelastase EC.3.4.21.37 |
| d) | Chymotrypsin EC 3.4.21.1 |
| e) | Pankreaselastase EC 3.4.21.36 |
| f) | Papain EC 3.4.22.2 |
| g + l) | Plasmin EC 3.4.21.7 |
| h + m) | Urokinase EC 3.4.21.31 |
| i) | Thiolproteinase:<br>Cathepsin B EC 3.4.22.1<br>Cathepsin L EC 3.4.22.15<br>Cathepsin H EC 3.4.22.16<br>Ficin EC 3.4.22.3<br>Bromalain EC 3.4.22.4 |
| j) | Leukocytenelastase EC 3.4.21.37<br>Cathepsin G EC 3.4.21.20 |
| k) | Plasmakallikrein EC 3.4.21.34 |
| n) | Trypsin EC 3.4.21.4 |
| o) | Thrombin EC 3.4.21.5 |

Als Laufmittel bei den weiter oben beschriebenen HPLC-Reinigungen dient eine Mischung aus

a) 0,05 Mol/l Ammoniumacetat in Wasser, pH = 8,0

b) Methanol

und zwar im Volumenverhältnis a:b von 40% :60% bis 50% :50% ; z.B. 42% :58% .

**Patentansprüche**

1. D-Aminoluciferine der allgemeinen Formel (I):

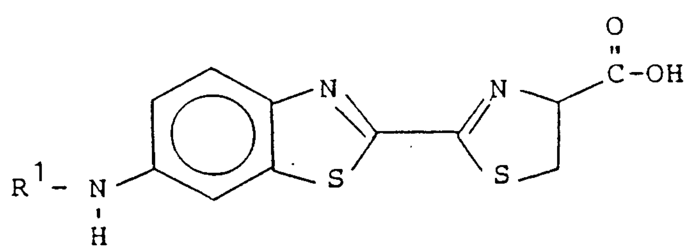

(I)

worin

R[1] einen L-Aminosäure- oder Peptidrest mit bis zu 10 L-Aminosäureeinheiten, der über die (endständige) Carboxygruppe als Amid gebunden ist und dessen freie Aminogruppen gegebenenfalls durch eine übliche Schutzgruppe geschützt sind, oder einen Monosaccharid- oder Disaccharidrest bedeutet.

2. Verbindungen nach Anspruch 1, worin die Aminosäurereste bzw. -einheiten von natürlich vorkommenden Aminosäuren stammen.

**3.** Verbindungen nach Anspruch 1, worin der Aminosäurerest $R^1$ der allgemeinen Formel (II)

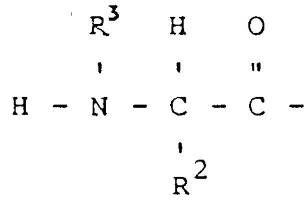

(II)

entspricht, worin

$R^3$ ein Wasserstoffatom oder eine übliche Schutzgruppe darstellt und

$R^2$ für -H, $-CH_3$, $-CH_2OH$, $-CH(OH)CH_3$, $-CH(CH_3)_2$, $-CH_2CH(CH_3)_2$, $-CH(CH_3)CH_2CH_3$ , $-CH_2COOH$, $-CH_2CONH_2$, $-CH_2CH_2COOH$, $-CH_2CH_2CONH_2$, $-CH_2CH_2CH_2CH_2NH_2$, $-CH_2C_6H_4OH$, $-CH_2C_6H_5$,

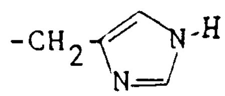 , 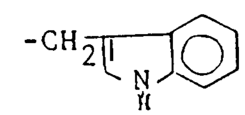 ,

$-CH_2SH$, $-CH_2CH_2CH_2$-NH-C(=NH)-$NH_2$ oder $-CH_2CH_2SCH_3$ steht, oder worin die Gruppe der allgemeinen Formel (II) ein entsprechender Prolin- oder Hydroxyprolinrest ist.

**4.** Verbindungen nach Anspruch 3, worin $R^2$ für $-CH_2C_6H_5$, $-CH_2CH_2CH_2$-NH-C(=NH)-$NH_2$, $-CH_2C_6H_4OH$, $-CH_2CH_2CH_2CH_2NH_2$ oder $-CH_3$ steht.

**5.** Verbindungen der allgemeinen Formel (I) gemäß Anspruch 1, worin $R^1$ einen Peptidrest mit bis zu 5 Aminosäureeinheiten gemäß den Ansprüchen 2 bis 4 bedeutet.

**6.** Verbindungen der allgemeinen Formel (I) gemäß einem der vorhergehenden Ansprüche, worin die freie(n) Aminogruppe(n) des Restes $R^1$ durch eine übliche Schutzgruppe geschützt ist (sind).

**7.** Verbindungen der allgemeinen Formel (I) gemäß Anspruch 6, dadurch gekennzeichnet, daß die Schutzgruppe eine Acetyl-, Benzoyl-, Tosyl-, Benzyloxycarbonyl-, tert.-Butyloxycarbonyl-, Succinyl- oder Methoxysuccinylgruppe ist.

**8.** Verfahren zur Herstellung der D-Aminoluciferine der allgemeinen Formel (I) gemäß einem der Ansprüche 1 bis 7, dadurch gekennzeichnet, daß man

a) zur Herstellung der Verbindungen der allgemeinen Formel (I), worin $R^1$ einen wie zuvor definierten L-Aminosäure- oder Peptidrest bedeutet, eine (ein ) dem zuvor definierten Aminosäure- bzw. Peptidrest entsprechende(s) N-geschützte(s) Aminosäure bzw. Peptid

entweder mit 2-Cyano-6-aminobenzothiazol der Formel (III)

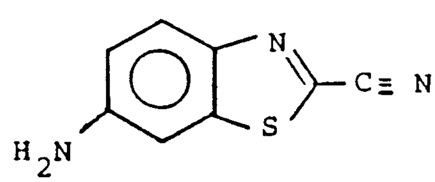

(III)

zu einer Verbindung der allgemeinen Formel (IV)

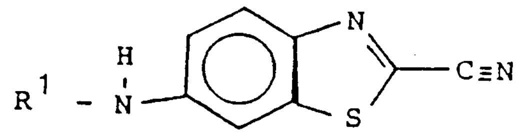

(IV)

umsetzt, worin $R^1$ die oben angegebenen Bedeutungen besitzt, und

die erhaltene Verbindung der allgemeinen Formel (IV) mit D-Cystein zu einer Verbindung der allgemeinen Formel (I) umsetzt und
gegebenenfalls die Aminoschutzgruppe(n) auf übliche Weise abspaltet

oder

mit D-Aminoluciferin, dessen Carboxylgruppe durch eine übliche Schutzgruppe geschützt ist, der Formel (Va)

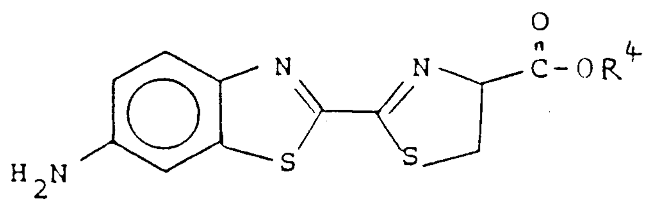

(Va)

worin

$R^4$ eine übliche Carboxylschutzgruppe bedeutet, umsetzt und

die Carboxylschutzgruppe sowie gegebenenfalls die Aminoschutzgruppe(n) auf übliche Weise entfernt,

oder
b) zur Herstellung einer Verbindung der allgemeinen Formel (I), worin $R^1$ einen Monosaccharid- oder Disaccharidrest bedeutet,

Aminoluciferin der Formel (Va), dessen Carboxylschutzgruppe geschützt ist, mit dem entsprechenden $Br^1$-Mono- oder Disaccharid umsetzt und anschließend die Schutzgruppe der Carboxylgruppe abspaltet.

**9.** Verwendung der Verbindungen der allgemeinen Formel (I) gemäß den Ansprüchen 1 bis 7 sowie von D-Aminoluciferin in Tests zur "in vitro" Bestimmung von Enzymaktivitäten.

**10.** Zusammensetzungen zur Bestimmung von Enzymaktivitäten, dadurch gekennzeichnet, daß sie mindestens ein Aminoluciferin nach einem der Ansprüche 1 bis 7 enthalten.

## Claims

**1.** D-Aminoluciferins of the general formula (I):

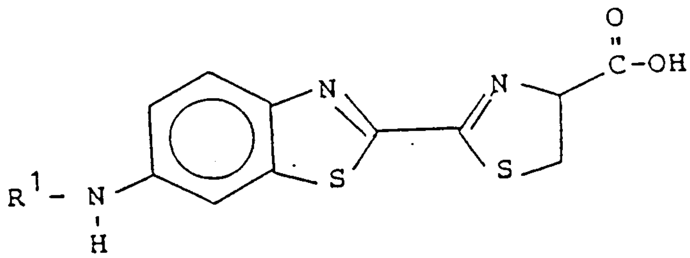

(I)

wherein
$R^1$ represents an L-amino acid or peptide radical having up to 10 L-amino acid units, said radical being bound via the (terminal) carboxyl group in the form of an amide and the free amino group of which may be protected by a common protective group, or a monosaccharide or disaccharide radical.

**2.** Compounds according to claim 1 wherein the amino acid radicals or units, respectively, are derived from naturally occurring amino acids.

**3.** Compounds according to claim 1 wherein the amino acid radical $R^1$ has the general formula (II)

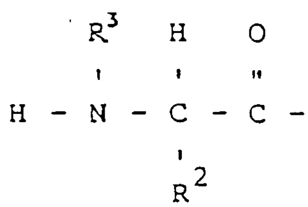

(II)

wherein
$R^3$ represents a hydrogen atom or a common protective group and
$R^2$ is -H, $-CH_3$, $-CH_2OH$, $-CH(OH)CH_3$, $-CH(CH_3)_2$, $-CH_2CH(CH_3)_2$, $-CH(CH_3)CH_2CH_3$, $-CH_2COOH$, $-CH_2CONH_2$, $-CH_2CH_2COOH$, $-CH_2CH_2CONH_2$, $-CH_2CH_2CH_2CH_2NH_2$, $-CH_2C_6H_4OH$, $CH_2C_6H_5$,

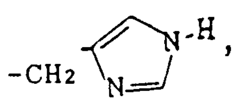 , 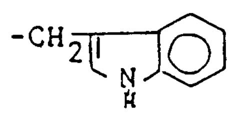 ,

$-CH_2SH$, $-CH_2CH_2CH_2-NH-C(=NH)-NH_2$ or $-CH_2CH_2SCH_3$ or wherein the group of the general formula (II) is a corresponding prolin or hydroxy prolin radical.

**4.** Compounds according to claim 3 wherein $R^2$ is $-CH_2C_6H_5$, $-CH_2CH_2CH_2-NH-C(=NH)-NH_2$, $-CH_2C_6H_4OH$, $-CH_2CH_2CH_2CH_2NH_2$ or $-CH_3$.

**5.** Compounds of the general formula (I) according to claim 1 wherein $R^1$ is a peptide radical having up to 5 amino acid units according to claims 2 to 4.

**6.** Compounds of the general formula (I) according to one of the preceeding claims wherein the free amino group(s) of the radical $R^1$ is (are) protected by a common protective group.

**7.** Compounds of the general formula (I) according to claim 6,
**characterized** in that
the protective group is an acetyl, benzoyl, tosyl, benzyloxycarbonyl, tert.-butyloxycarbonyl, succinyl or methoxysuccinyl group.

**8.** Process for preparing the D-aminoluciferins of the general formula (I) according to one of the claims 1 to 7,
**characterized** in that

a) for producing the compounds of the general formula (I), wherein $R^1$ represents one of the above-mentioned L-amino acid or peptide radicals, an N-protected amino acid or peptide, respectively, corresponding to one of the above defined amino acid or peptide radicals, respectively, is reacted with
either a 2-cyano-6-aminobenzothiazole of the formula (III)

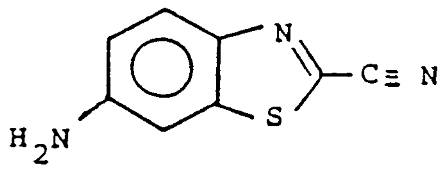
(III)

to give a compound of the general formula (IV)

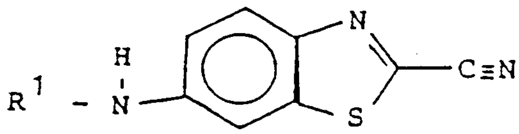
(IV)

wherein
$R^1$ has the meanings given above and
the thus obtained compound of the general formula (IV) is reacted with D-cysteine to give a compound of the general formula (I) and
the amino protective group(s) are, if desired, cleaved off in common manner
or
with D-aminoluciferin the carboxyl group of which is protected by a common protective group and which has the formula (Va)

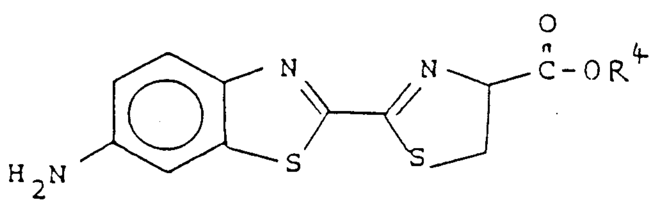
(Va)

wherein
$R^4$ represents a common protective group for a carboxyl group and
the protective group of the carboxyl group as well as the protective group(s) for the amino group(s) are removed in a common manner,
or
b) for producing a compound of the general formula (I), wherein $R^1$ represents a monosaccharide or disaccharide radical, the aminoluciferin of the formula (Va), the carboxyl group of which is protected, is reacted with the corresponding $Br^1$-mono or disaccharide and the protective group of the carboxyl group is then cleaved off.

**9.** Use of the compounds of the general formula (I) according to claims 1 to 7 as well as of D-aminoluciferin in tests for the "in vitro" determination of enzyme activities.

**10.** Compositions for determining enzyme activities
**characterized** in that
they contain at least one aminoluciferin according to one of the claims 1 to 7.

## Revendications

**1.** D-aminolucifèrines de formule générale (I)

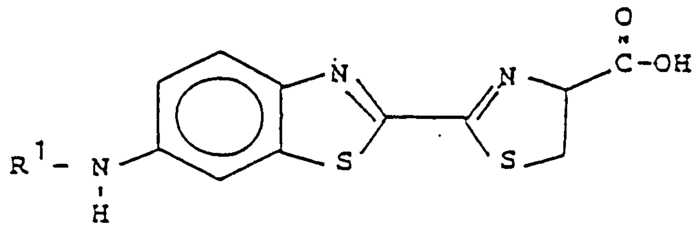

(I)

dans laquelle

$R^1$ représente un reste L-aminoacide ou peptide ayant jusqu'à 10 motifs L-aminoacide, qui est lié sous forme d'amide par l'intermédiaire de groupes carboxyle (terminaux) et dont le ou les groupes amino libres sont éventuellement protégés par un groupe de protection usuel, ou représente un reste monosaccharide ou disaccharide.

**2.** Composés selon la revendication 1, dans lesquels les restes ou motifs aminoacide proviennent d'aminoacides naturels.

**3.** Composés selon la revendication 1, dans lesquels le reste aminoacide répond de préférence à la formule générale (II)

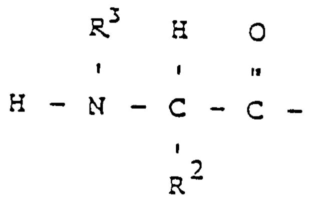

(II)

dans laquelle

$R^3$ représente un atome d'hydrogène ou un groupe de protection usuel, et

$R^2$ représente

$-H$, $-CH_3$, $-CH_2OH$, $-CH(OH)CH_3$, $-CH(CH_3)_2$, $-CH_2CH(CH_3)_2$, $-CH(CH_3)CH_2CH_3$ , $-CH_2COOH$, $-CH_2CONH_2$, $-CH_2CH_2COOH$, $-CH_2CH_2CONH_2$, $-CH_2CH_2CH_2CH_2NH_2$, $-CH_2C_6H_4OH$, $-CH_2C_6H_5$,

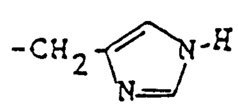 , 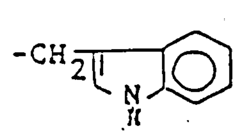 ,

$-CH_2SH$, $-CH_2CH_2CH_2-NH-C(=NH)-NH_2$

ou $-CH_2CH_2SCH_3$,

ou dans lesquels le groupe de formule générale (II) est un reste proline ou hydroxyproline approprié.

**4.** Composés selon la revendicaton 3, dans lesquels $R^2$ représente un groupe $-CH_2C_6H_5$, $-CH_2CH_2CH_2-NH-C(=NH)-NH_2$,$-CH_2C_6H_4OH$, $-CH_2CH_2CH_2CH_2NH_2$ ou $-CH_3$.

**5.** Composés de formule générale (I) selon la revendication 1, dans lesquels $R^1$ représente un reste peptide comportant jusqu'à 5 motifs aminoacide, selon les revendications 2 à 4.

**6.** Composés de formule générale (I) selon l'une quelconque des revendications précédentes, dans lesquels le ou les groupe(s) amino libre(s) des restes $R^1$ est ou sont protégés par un groupe protecteur

usuel.

7. Composés de formule générale (I) selon la revendication 6, caractérisés en ce que le groupe protecteur est un groupe acétyle, benzoyle, tosyle, benzyloxycarbonyle, tert.-butyloxycarbonyle, succinyle ou méthoxysuccinyle.

8. Procédé pour la préparation de la D-aminoluciférine de formule générale (I), selon l'une quelconque des revendications 1 à 7, caractérisé en ce que
   a) pour la préparation des composés de formule générale (I), dans laquelle $R^1$ représente un reste L-aminoacide ou peptide défini précédemment,

   on fait réagir un reste aminoacide ou peptide N-protégé correspondant au reste aminoacide ou peptide défini précédemment,

   soit avec le 2-cyano-6-aminobenzothiazol de formule (III)

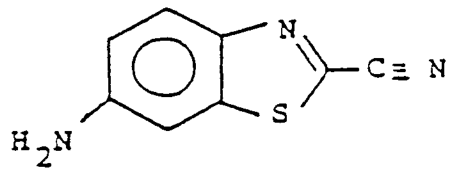

(III)

   pour donner un composé de formule générale (IV)

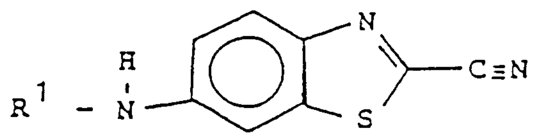

(IV)

   dans laquelle $R^1$ a la signification mentionnée ci-dessus, et on fait réagir le composé obtenu de formule générale (IV) avec la D-cystéine pour donner un composé de formule générale (I), et

   on sépare éventuellement le ou les groupe(s) aminoprotecteur(s), de la manière habituelle,

   soit

   avec l'aminoluciférine, dont le groupe carboxyle est protégé par un groupe protecteur usuel, de formule (Va)

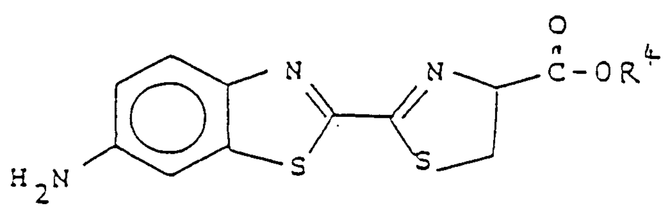

(Va)

dans laquelle
   $R^4$ représente un groupe carboxyle-protecteur usuel, et

   on élimine le groupe carboxyle-protecteur ainsi qu'éventuellement le ou les groupe(s) aminoprotecteur(s) de la manière habituelle, ou
   b) pour la préparation d'un composé de formule générale (I), dans laquelle $R^1$ représente un reste monosaccharide ou disaccharide,
   on fait réagir une aminoluciférine de formule (Va), dont le groupe carboxyle est protégé, avec le

$Br^1$-mono- ou disaccharide, et ensuite, on sépare le groupe protecteur du groupe carboxyle.

**9.** Utilisation des composés de formule générale (I) selon les revendications 1 à 7, ainsi que de D-aminoluciférine, dans des tests pour la détermination "in vitro" d'activités enzymatiques.

**10.** Compositions pour la détermination d'activités enzymatiques, caractérisées en ce qu'elles contiennent au moins une aminoluciférine selon l'une des revendications 1 à 7.